# EUROPÄISCHE PATENTSCHRIFT

(11) **EP 1 022 024 B1**
(45) Veröffentlichungstag und Bekanntmachung des Hinweises auf die Patenterteilung: **20.07.2005**
(21) Anmeldenummer: 00101455.4
(22) Anmeldetag: 25.01.2000
(51) Int. Cl.: A61K 31/352, A61P 1/16

(54) **Anticholestatische Wirksamkeit von Luteolin, Apigenin und Naringenin**
Anticholestatic activity of luteolin, apigenin and naringenin
Activité anticholestasique de la lutéoline, apigénine et naringénine

(30) Priorität: 25.01.1999 DE 19902773
(43) Veröffentlichungstag der Anmeldung: 26.07.2000
(73) Patentinhaber: Sertürner Arzneimittel GmbH, 33332 Gütersloh (DE)
(72) Erfinder: Gebhardt, Rolf, 04103 Leipzig (DE)
(74) Vertreter: Huber, Bernhard, Dipl.-Chem.

(56) Entgegenhaltungen:
- EP-A- 0 807 435
- FINTELMANN V: "ANTIDYSPEPTISCHE UND LIPID-SENKENDE WIRKUNGEN VON ARTISCHOCKENEXTRAKT" ZFA. ZEITSCHRIFT FUER ALLGEMEINMEDIZIN, HIPPOKRATES VERLAG, STUTTGART, DE, Bd. 72, 1996, Seiten 48-57, XP001027358 ISSN: 0341-9835
- KRAFT K: "ARTICHOKE LEAF EXTRACT - RECENT FINDINGS REFLECTING EFFECTS ON LIPID METABOLISM, LIVER AND GASTROINTESTINAL TRACTS" PHYTOMEDICINE, GUSTAV FISCHER VERLAG, STUTTGART, DE, Bd. 4, Nr. 4, 1997, Seiten 369-378, XP008000233 ISSN: 0944-7113
- DATABASE CA [Online] CHEMICAL ABSTRACTS SERVICE, COLUMBUS, OHIO, US; PROKOPENKO, O. P. ET AL: "Phenol compounds of Helichrysum and their biological activity" retrieved from STN Database accession no. 78:52637 XP002234971 & FARMATSEVTICHNII ZHURNAL (KIEV) (1972), 27(4), 3-7 ,
- DJERABA C ET AL: "A RETRIEVAL BY CONTENT SYSTEM IN A DATABASE" ACTES DU CONGRES INFORSID - PROCEEDINGS OF THE INFORSID CONGRESS, XX, XX, 10. Juni 1997 (1997-06-10), Seiten 507-525, XP000900681
- LISEVITSKAYA L. ET AL.: "CHEMICAL ABSTRACTS + INDEXES, AMERICAN CHEMICAL SOCIETY. COLUMBUS, US" CHEMICAL ABSTRACTS + INDEXES, AMERICAN CHEMICAL SOCIETY. COLUMBUS, US, Bd. 76, Nr. 19, 8. Mai 1972 (1972-05-08), Seite 37 XP002900217 ISSN: 0009-2258
- DATABASE CA [Online] CHEMICAL ABSTRACTS SERVICE, COLUMBUS, OHIO, US; PAGANI, F. ET AL: "Flavonoids for Melampyrum nemorosum (Scrophulariaceae)" retrieved from STN Database accession no. 77:45556 XP002234972 & BOLLETTINO CHIMICO FARMACEUTICO (1971), 110(12), 695-703 ,

## Beschreibung

Die Erfindung betrifft ein Verfahren zur Herstellung eines Arzneimittels zur Behandlung oder Prävention einer intrahepatischen Cholestase.

Bei Cholestase handelt es sich um ein Syndrom, das bei gestörtem Gallefluss entsteht. Man unterscheidet extrahepatische und intrahepatische Cholestase. Die extrahepatische Cholestase wird meist durch einen Gallengangstein oder ein Pankreaskarzinom hervorgerufen. Bei intrahepatischer Cholestase handelt es sich um eine Stauung in den Gallengängen. Die häufigsten intrahepatischen Ursachen sind virale und andere Hepatitiden, Medikamente, z.B. zur Krebsbehandlung, sowie die alkoholinduzierte Leberschädigung. Weniger häufige Ursachen sind die primäre billiäre Zirrhose, die Cholestase in der Schwangerschaft, und weitere Erkrankungen.

Extrakt aus Blättern der Artischocke (Cyanara scolymus L.) ist für seine choleretische Wirkung bei dyspeptischen Beschwerden bekannt. Echte choleretische Mittel, als welches ein Artischockenextrakt angesehen wird, führen zu einer Erhöhung der Menge an Gallensäuren, wohingegen unechte choleretische Mittel zu einer Erhöhung des Volumens an Gallenflüssigkeit führen. Neben der choleretischen Wirkung wird für Artischockenextrakt in der Literatur auch eine anticholestatische Wirkung genannt (Ärzte-Ztg., 15. Jahrgang, 1996, Seite 16). Allerdings war bisher nicht bekannt, welcher Wirkstoff die anticholestatische Wirkung hervorruft.

Aufgabe der vorliegenden Erfindung war es deshalb, ein Arzneimittel bereitzustellen, das einen Wirkstoff mit anticholestatischer Wirkung mit definierter Wirksamkeit enthält.

Diese Aufgabe wird erfindungsgemäß gelöst durch die Verwendung mindestens einer Verbindung der allgemeinen Formel I 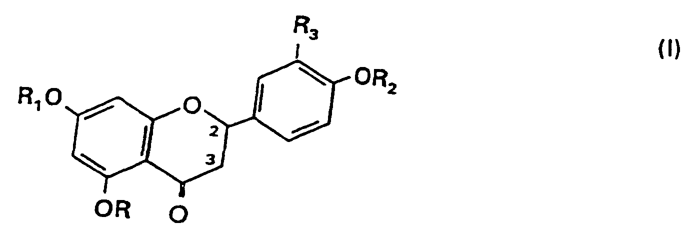 worin R, R₁ und R₂ unabhängig voneinander jeweils = H, C₁₋₂-Alkyl oder C₁₋ ₃-Acyl, R₃ = H oder OR₄ und R₄ = H, C₁₋₂-Alkyl oder C₁-C₄-Acyl bedeuten und zwischen den Positionen 2 und 3 eine Einfach- oder Doppelbindung vorliegt, oder eines physiologisch annehmbaren Salzes davon als Wirkstoff zur Herstellung eines Arzneimittels zur Behandlung oder Prävention einer intrahepatischen Cholestase.

Es wurde gefunden, dass die Verbindungen der Formel I eine anticholestatische Wirksamkeit aufweisen und deshalb zur Behandlung einer intrahepatischen Cholestase geeignet sind. Eine intrahepatische Veränderung der Gallengänge kann mit Verbindungen der Formel I verhindert oder rückgängig gemacht werden, sodass auch eine prophylaktische Behandlung zur Prävention einer intrahepatischen Cholestase möglich ist.

Durch die Kenntnis, dass die Verbindungen der Formel I eine anticholestatische Wirksamkeit aufweisen, ist die Herstellung eines Arzneimittels mit vorbestimmter und definierter Wirkungsdosis und eine Standardisierung möglich. Weiterhin kann mit der erfindungsgemäßen Verwendung eine schnelle und zielgerichtete Wirkung erreicht werden.

In Artischockenextrakten, insbesondere Artischockenblätter-Trockenextrakten, wie sie beispielsweise kommerziell von Sertürner Arzneimittel unter dem Namen HEPAR-SL® forte erhältlich sind, liegen zahlreiche Verbindungen vor, bei denen es sich um mögliche Wirkstoffe handeln könnte. Die anticholestatische Wirksamkeit konnte bisher keiner der zahlreichen im Extrakt vorliegenden Verbindungen zugeordnet werden. Es war somit schwierig, aus dem Artischockenextrakt ein standardisiertes Arzneimittel herzustellen.

Es wurde nun festgestellt, dass gerade die Verbindungen der Formel I eine anticholestatische Wirksamkeit aufweisen. Freie Verbindungen der Formel I, insbesondere Luteolin, liegen, wenn überhaupt, nur in geringen Mengen von < 0,08 Gew.-%, bezogen auf den Gesamtextrakt, im Artischockenextrakt vor. Zudem findet sich im Stand der Technik kein Hinweis, dass gerade Verbindungen der Formel I eine anticholestatische Wirkung aufweisen könnten.

Bekannte Bestandteile von Artischockenextrakt sind Kaffeesäure, Chlorogensäure, Cynarin(1,5-di-O-Caffeoyl-chinasäure), 1,3-di-O-Caffeoylchinasäure, Scolymosid und Cynarosid (Luteolin-7-O-glucosid). Cynarosid (Luteolin-7-O-glucosid) wird im Magen-Darm-Trakt gespalten, wobei Luteolin freigesetzt wird. Allerdings findet sich Cynarosid auch in vielen anderen Pflanzen, ohne dass von solchen eine anticholestatische Wirksamkeit berichtet worden ist. Cynarosid selbst hat keine anticholestatische Wirkung.

Die Wirkmechanismen von anticholestatischen und choleretischen Mitteln sind grundsätzlich verschieden. Eine häufig als Modell verwendete Cholestase, die durch Taurolithocholat ausgelöst wird, bewirkt eine leicht detektierbare Membranveränderung. Bekannte choleretische Substanzen sind im allgemeinen nicht in der Lage, diese Membranveränderung zu verhindern oder zu beseitigen (Layden et al., Gastroenterology 50 (1977), 2305 bis 2312. Z.B. zeigte die als choleretisch wirksam bekannte Dehydrocholsäure bei einer durch Taurolithocholat ausgelösten Cholestase keine Wirksamkeit, sodass eine Verwendung von choleretisch wirksamen Verbindungen bei einer Cholestase im allgemeinen nicht angezeigt ist.

Bei Verbindungen der allgemeinen Formel I, in denen R, R, oder/und R₂ H darstellen, liegt an den entsprechenden Positionen 5, 7 bzw. 4' des Flavon-bzw. Flavanon-Grundgerüstes eine Hydroxylgruppe vor. Für den Fall, dass es sich bei R, R₁ oder/und R₂ um eine C₁₋₂-Alkyl-, insbesondere Methyl- oder Ethyl-, besonders bevorzugt eine Methylgruppe handelt, liegt an den entsprechenden Positionen eine Ethergruppierung vor. Falls R, R₁ oder/und R₂ eine C₁₋₃-Acylgruppe ist, liegt an den entsprechenden Positionen eine Estergruppe vor. Bevorzugte Acylgruppierungen sind ein Formylrest, ein Acetylrest, ein Propionylrest und ein Lactylrest. Verbindungen, bei denen zwischen den Positionen 2 und 3 eine Einfachbindung vorliegt, weisen ein Flavanongrundgerüst 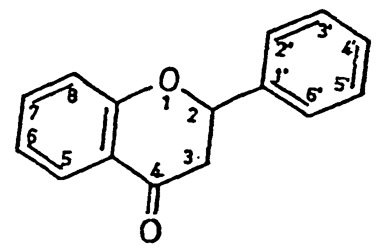 auf, Verbindungen, bei denen zwischen den Positionen 2 und 3 eine Doppelbindung vorliegt, weisen ein Flavongrundgerüst 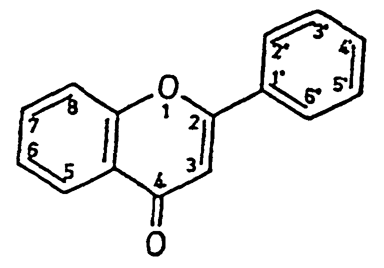 auf.

Ein physiologisch annehmbares Salz einer Verbindung der Formel I ist jede salzartige Verbindung, die die Verbindung der Formel I in ionischer Form zusammen mit einem Gegenion umfasst, welches physiologisch unbedenklich ist. Beispiele solcher Salze sind insbesondere Säureadditionssalze.

Bevorzugt werden zur Herstellung eines Arzneimittels zur Behandlung oder Prävention einer intrahepatischen Cholestase Verbindungen der allgemeinen Formel I, worin R, R₁ und/oder R₂ = H verwendet sind. Es wurde gefunden, dass solche Verbindungen, in denen freie OH-Gruppen, insbesondere an den Positionen 5 (entspricht Rest R) und/oder 7 (entspricht Rest R₁) vorliegen, eine besonders starke Wirksamkeit aufweisen.

Weiterhin bevorzugt werden Verbindungen der allgemeinen Formel I, worin R₂ eine OH-Gruppe darstellt, verwendet.

Eine besonders hohe Wirksamkeit wurde bei Luteolin (R = H, R, = H, R₂ = H, R₃ = OH, wobei zwischen den Positionen 2 und 3 eine Doppelbindung vorliegt), Apigenin (R = H, R₁ = H, R₂ = H, R₃ = H, wobei zwischen den Positionen 2 und 3 eine Doppelbindung vorliegt) und Naringenin (R = H, R₁ = H, R₂ = H, R₃ = H, wobei zwischen den Positionen 2 und 3 eine Einfachbindung vorliegt) gefunden, wobei Luteolin am meisten bevorzugt ist. Die Verbindungen Luteolin-7-O-glucosid, 3',4'-Hydroxyflavon, Genistein und Daidzein zeigten keine anticholestatische Wirkung.

Die Verbindungen der Formel I werden zur Herstellung eines Arzneimittels zur Behandlung oder Prävention einer intrahepatischen Cholestase bevorzugt in einer Menge von ≥ 0,1 Gew.-%, mehr bevorzugt ≥ 1 Gew.-%, besonders bevorzugt ≥ 10 Gew.-% und am meisten bevorzugt ≥ 20 Gew.-% verwendet. Es ist aber auch möglich, einen noch höheren Gehalt an mindestens einer Verbindung der Formel I zu verwenden, insbesondere ≥ 50 Gew.-%, bezogen auf das Gesamtgewicht des Arzneimittels.

Für eine wirksame Behandlung oder Prävention einer intrahepatischen Cholestase werden einem Patienten im allgemeinen von 10 mg bis 1000 mg einer Verbindung der allgemeinen Formel I/ pro Tag verabreicht. Bevorzugt werden mindestens 20 mg, insbesondere mindestens 50 mg und besonders bevorzugt mindestens 100 mg einer Verbindung der allgemeinen Formel I pro Tag pro Person und höchstens bis zu 750 mg, bevorzugt bis zu 500 mg, besonders bevorzugt bis zu 300 mg pro Person und Tag verabreicht.

Zur Herstellung eines Arzneimittels können gegebenenfalls physiologisch verträgliche Hilfs- und Trägerstoffen vorformuliert werden. Solche Hilfsstoffe müssen physiologisch unbedenklich sein und dürfen die Wirksamkeit der Wirkstoffe nicht nachteilig beeinflussen. Geeignete Hilfsstoffe sind dem Fachmann bekannt und umfassen beispielsweise Füllstoffe, Bindemittel und Gleitmittel, wie etwa mikrokristalline Cellulose, Amylose, Lactose, Mannit, Talkum, Magnesiumstearat, Stärke, hochdisperses Magnesiumoxid, Siliciumdioxid, Natriumcarboxymethylstärke, Polyvidon, Macrogol und andere.

Mit Hilfe der vorliegenden Erfindung können alle, dem Fachmann bekannten verabreichbaren Arzneimittelformen hergestellt werden. Bevorzugt wird eine Formulierung hergestellt, die zur oralen Verabreichung geeignet ist, insbesondere in fester Form. Beispiele solcher fester Arzneimittelformen umfassen Kapseln, Tabletten, Granulate, Dragees, Filmtabletten und ähnliches. Bevorzugt sind Formulierungen, bei denen der Wirkstoff erst im Magen-Darm-Trakt freigesetzt wird.
Zur Herstellung eines Arzneimittels zur Behandlung oder Prävention einer intrahepatischen Cholestase kann eine einzige wirksame Verbindung der allgemeinen Formel I oder mehrere solcher wirksamen Verbindungen verwendet werden. Es kann dabei ein Arzneimittel hergestellt werden, in dem mindestens eine Verbindung der allgemeinen Formel I der einzige Wirkstoff ist, d.h. das Arzneimittel enthält neben den Verbindungen der allgemeinen Formel I lediglich inerte Hilfs- und Trägerstoffe. Zur Herstellung eines solchen Arzneimittels können Wirkstoffe verwendet werden, die aus pflanzlichen Extrakten isoliert wurden, ebenso wie chemisch synthetisierte Verbindungen. Die Isolierung von entsprechenden Flavon- oder Flavanonderivaten, beispielsweise Luteolin aus pflanzlichen Extrakten ist in der Literatur ausführlich beschrieben (vgl. z.B. Pharmacia 21 (39) (1972) 37). Die chemische Synthese der Verbindungen der allgemeinen Formel I kann ebenfalls nach an sich bekannten und dokumentierten Verfahren durchgeführt werden (vgl. z.B. J. Chem. Soc. 1939, 91). Darüber hinaus sind zahlreiche Verbindungen der Formel I kommerziell erhältlich.

Daneben ist es auch möglich, ein Arzneimittel herzustellen, das neben der Verbindung der allgemeinen Formel I weitere Wirkstoffe enthält. Hierzu kann eine isolierte oder chemisch hergestellte Verbindung der Formel I mit weiteren Wirkstoffen und gegebenenfalls Hilfs- oder Trägerstoffen gemischt werden. Daneben ist es auch möglich, einen Pflanzenextrakt zu verwenden, beispielsweise einen Artischockenextrakt, der neben den Verbindungen der allgemeinen Formel I weitere Wirkstoffe enthält. Im Fall der Verwendung eines natürlichen Extraktes wird ein Arzneimittel dadurch hergestellt, dass ein herkömmlicher Extrakt an Verbindungen der Formel I angereichert wird, sodass er ≥ 0,1 Gew.-%, bevorzugt ≥ 1 Gew.-%, mehr bevorzugt ≥ 10 Gew.-% und am meisten bevorzugt ≥ 20 Gew.-% an Verbindungen der Formel I, bezogen auf das Extraktgesamtgewicht, aufweist. Eine solche Anreicherung kann dadurch erfolgen, dass die Extraktionsbedingungen so gewählt werden, dass im Extrakt ein möglichst höher Gehalt an Verbindungen der allgemeinen Formel I vorliegt. Es ist aber auch möglich, einem handelsüblichen Extrakt einen zusätzlichen Gehalt an mindestens einer Verbindung der allgemeinen Formel I zuzugeben, um auf diese Weise eine Erhöhung der Wirkstoffkonzentration zu erzielen.

Eine weitere Möglichkeit, einen an Wirkstoff angereicherten Extrakt zu erhalten besteht darin, einen handelsüblichen Extrakt, beispielsweise einen Artischockenextrakt, vor der Verwendung durch Spaltung von glykosylierten Verbindungen der Formel I an freien Verbindungen der Formel I anzureichern. Eine solche Spaltung kann beispielsweise durch Hitze, pH-Wert oder enzymatisch erfolgen. So kann beispielsweise der Gehalt an Luteolin in einem Artischockenextrakt durch Spaltung des im Artischockenextrakt vorliegenden Cyanarosids in freies Luteolin vor der Verabreichung erzielt werden. Auf entsprechende Weise können Ester- und Ether-substituierte Verbindungen der allgemeinen Formel I beispielsweise durch entsprechend spaltende Enzyme in die wirksame Form überführt werden.

Die Erfindung wird durch das folgende Beispiel weiter erläutert.

### Beispiel 1

### Verhinderung einer Taurolithocholat-induzierten Transformation kanilikulärer Membranen

Taurolithocholat induziert in Ratten in vivo eine schwere Cholestase, die mit der Umwandlung der Gallekanälchen in bizarr strukturierte, lamelläre Membranformationen verbunden ist. Die Induzierung einer intrahepatischen Cholestase durch Lithocholsäure und Natriumtaurolithocholat in Tierversuchen ist ausführlich beschrieben (Javitt, N.B., Nature 210, (1966), 1262-1263; King. J.E. et al., J. Clin. Invest. 50, (1971) 2305-2312; Layden et al., Gastroenterology 73 (1977), 120-128; Mockel et al., Am. J. Physiol., 269 (1995), G73-G84). Neben einer kanalikulären Dilatation induzieren sie bizarre lamellare Deformationen der Ultrastruktur der Gallekanälchen, die charakteristisch für diese Art von Cholestase sind (Miyai et al., Lab. Invest. 32 (1975), 527-535; Kakis et al., Lab. Invest. 43, (1980) 73-81). Kürzlich wurde gezeigt, dass Taurolithocholat, das den Primärkulturen von Rattenhepatozyten zugegeben wurde, ähnliche Deformationen in vitro bildet, die von den in vivo gebildeten nicht unterscheidbar sind (Jung et al., Eur. J. Cell Biol. 29, (1982) 77-82; Thibault et al., J. Hepatol. 19 (1993) 367-376). Für die Versuche wurde deshalb in Primärkulturen von Rattenhepatocyten die Transformation neu gebildeter Gallekanälchen durch Taurolithocholat wie beschrieben (Jung et al., Eur. J. Cell Biol. 29 (1982) 77-82) in vitro ausgelöst.

Leberparenchymzellen wurden aus männlichen Sprague-Dawley-Ratten (220 bis 280 g) isoliert und für 2 Tage in einem serumfreien William-Medium E wie beschrieben kultiviert (Gebhardt et al., Cell Biol. Toxicol. 6 (1990) 369-372; Gebhardt, Toxicol. Appl. Pharmacol. 144 (1997), 270-286). Für die Behandlung der kultivierten Hepatocyten mit Taurolithocholat wurde eine 10 mM Vorratslösung dieses Gallensalzes mit Ethanol hergestellt, die mit Williams-Medium E verdünnt wurde, um eine Endkonzentration von 0,1 mM zu ergeben. Für 1 bis 2 Tage kultivierte Hepatocyten wurden mit dem Taurolithocholat enthaltenden Medium für 3 Stunden behandelt, um maximale Veränderungen der Gallekanälchen zu induzieren (Jung et al., Eur. J. Cell Biol. 29 (1982), 77-82). Taurolithocholat (0,1 mM) induziert an primär kultivierten Rattenhepatocyten innerhalb von 3 Stunden eine ausgeprägte Transformation der kanalikulären Membranen zu bizarr strukturierten Formationen. Die normale kanalikuläre Membran, die mit zahlreichen Mikrovilli bedeckt ist, wird in bizarre lamillare Strukturen transformiert, die fast die gesamten Kanälchen ausfüllen. Dieser Vorgang scheint nach 2 bis 3 Stunden vollständig abgelaufen zu sein und betraf etwa 50 bis 70 % der Kanälchen am zweiten Tag der Kultivierung.

Die Membranveränderungen im Bereich der Gallekanälchen wurden mittels Elektronenmikroskopie an Ultradünnschnitten nachgewiesen (vgl. Gebardt et al., Eur. J. Cell Biol. 29 (1982) 68-76). Dazu wurden kultivierte Zellen für 30 bis 45 Minuten in situ mit 2,5 % Glutaraldehyd in 0,1 M Cacodylatpuffer, pH 7,2 bei 0 °C fixiert. Nach Waschen mit Cacodylatpuffer wurden die Zellen in eine 2 %-ige Lösung von Agar bei 46 ° C gegeben, das in kleine Stücke geschnitten wurde und in 1 % OsO₄ in einem Cacodylatpuffer nachfixiert wurde. Nach Dehydration wurden die Blöcke in Epon 812 eingebettet. Silberschnitte wurden mit einem Siemens la-Elektronenmikroskop nach Anfärben untersucht.

Für eine halbquantitative Abschätzung der Veränderung der kanalikulären Membranen wurde die Erscheinungsform von 100 Gallekanälchen aus zwei bis drei verschiedenen Experimenten untersucht. Gallekanälchen wurden als transformiert eingestuft, wenn die Hälfte oder mehr der kanalikulären Fläche mit bizarren lamellaren Strukturen bedeckt war.

Der oben beschriebene Versuch wurde wiederholt, wobei den Primärkulturen der Rattenhepatocyten gleichzeitig mit dem Taurolithocholat verschiedene Wirkstoffe zugegeben wurden. Die Zugabe von Luteolin in einer Menge von 28 mg gleichzeitig mit Taurlithocholat ergab eine vollständige Hemmung der lamellaren Transformation. Statt dessen behielten die Gallenkanälchen ihre Mikrovilli, wenn auch in etwas geringerer Anzahl und erschienen nur leicht dillatiert. Eine vergleichbare Wirkung konnte erzielt werden, wenn die Kulturen mit Luteolin vorinkubiert wurden. Dies zeigt, dass eine mögliche Adsorption von Taurolithocholat an andere Inhaltsstoffe keine Rolle spielt. Vorzugsweise sollte jedoch zwischen der Vorinkubation mit dem Wirkstoff unter Einwirkung von Taurolithocholat nicht mehr als 60 Minuten vergehen.

Weiterhin wurde gefunden, dass der Schutz gegen Taurolithocholat induzierte Transformation dosisabhängig war.

Die Versuche wurden mit Apigenin, einem Gentiana lutea Extrakt und dem Isoflavonoid Genistein wiederholt. Während Apigenin dieselben Wirkungen wie Luteolin hervorrief, waren der Gentiana lutea Extrakt und das Isoflavonoid Genistein wirkungslos.

Die Verbindungen der Formel I sind somit in der Lage, die Taurolithocholatinduzierte Umwandlung der kanalikulären Membranen spezifisch zu verhindern und anticholestatisch zu wirken. Die Wirkung wird vermutlich über die Hemmung von Proteinkinasen hervorgerufen.

## Patentansprüche

1. Verwendung mindestens einer Verbindung der allgemeinen Formel I 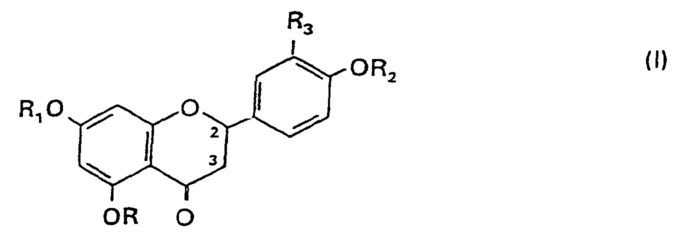 worin R, R₁ und R₂ unabhängig voneinander jeweils = H, C₁₋₂-Alkyl oder C₁₋₃-Acyl, R₃ = H oder OR₄ und R₄ = H, C₁₋₂-Alkyl oder C₁-C₄-Acyl bedeuten und zwischen den Positionen 2 und 3 eine Einfach- oder Doppelbindung vorliegt, oder eines physiologisch annehmbaren Salzes davon als Wirkstoff zur Herstellung eines Arzneimittels zur Behandlung oder Prävention einer intraheptischen Cholestase.

2. Verwendung nach Anspruch 1,
**dadurch gekennzeichnet, dass**
R, R₁ und R₂ = H.

3. Verwendung nach Anspruch 1 oder 2,
**dadurch gekennzeichnet, dass**
R₃ = OH.

4. Verwendung nach einem der Ansprüche 1 bis 3,
**dadurch gekennzeichnet, dass**
das Arzneimittel einen Gehalt an Verbindungen der allgemeinen Formel I von ≥ 1 Gew.-% aufweist.

5. Verwendung nach einem der Ansprüche 1 bis 3,
**dadurch gekennzeichnet, dass**
das Arzneimittel einen Gehalt an Verbindungen der allgemeinen Formel I von ≥ 20 Gew.-% aufweist.

## Claims

1. Use of at least one compound having the general formula I, 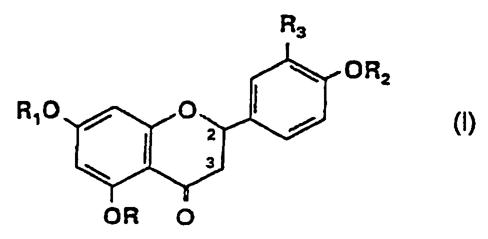 where R, R₁ and R₂ are independent of each other and can each = H, C₁₋₂-alkyl or C₁₋₃-acyl, R₃ = H or OR₄ and R₄ = H, C₁₋₂-alkyl or C₁₋₃-acyl, and there is a single or double bond between positions 2 and 3, or of a physiologically acceptable salt thereof as active agent for the manufacture of a drug for treating or preventing mtrahepatic cnotesiases.

2. Use according to claim 1,
**characterised in that**
R, R₁ and R₂ = H.

3. Use according to claim 1 or 2,
**characterised in that**
R₃ = OH.

4. Use according to one of the claims 1 to 3,
**characterised in that**
the drug contains compounds with the general formula I in a quantity of ≥ 1 wt%.

5. Use according to one of the claims 1 to 3,
**characterised in that**
the drug contains compounds with the general formula I in a quantity of ≥ 20 wt %.

## Revendications

1. Utilisation d'au moins un composé de formule générale I 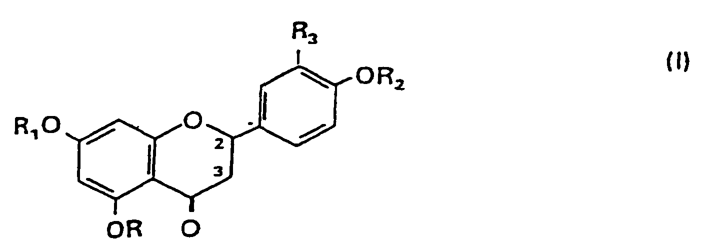 où R, R₁ et R₂ représentent indépendamment les uns des autres dans chaque cas H, C₁₋₂₋alkyle ou C₁₋₃-acyle, R₃ = H ou OR₄ et R₄ = H, C₁₋₂-alkyle ou C₁₋₄-acyle et une simple ou double liaison existe entre les positions 2 et 3, ou d'un sel physiologiquement acceptable de celui-ci comme principe actif pour la production d'un médicament pour le traitement ou la prévention d'une cholestase intrahépatique.

2. Utilisation selon la revendication 1 **caractérisée en ce que** R, R₁ et R₂ = H.

3. Utilisation selon la revendication 1 ou 2 **caractérisée en ce que** R₃ = OH.

4. Utilisation selon l'une des revendications 1 à 3 **caractérisée en ce que** le médicament présente une teneur en composés de formule générale I ≥ 1 % en masse.

5. Utilisation selon l'une des revendications 1 à 3 **caractérisée en ce que** le médicament présente une teneur en composés de formule générale I ≥ 20 % en masse.
